# EUROPÄISCHE PATENTANMELDUNG

(11) **EP 3 593 792 A1**
(43) Veröffentlichungstag der Anmeldung: **15.01.2020**
(21) Anmeldenummer: 18182887.2
(22) Anmeldetag: 11.07.2018
(51) Int. Cl.: A61K 9/20, A61K 31/407, A61P 19/02

(54) **PHARMAZEUTISCHE ZUSAMMENSETZUNG UMFASSEND 6-(4-CHLORPHENYL)-2,2-DIMETHYL-7-PHENYL-2,3-DIHYDRO-1H-PYRROLIZIN-5-YL-ESSIGSÄURECHOLINSALZ**

(71) Anmelder: Welding GmbH & Co. KG, 20354 Hamburg (DE)
(72) Erfinder: KEMKEN, Jens, 22455 Hamburg (DE); FRIEBEL, Christian, 22419 Hamburg (DE); HAMANN, Petra, 21423 Winsen (DE)
(74) Vertreter: Hoffmann Eitle

(57) **Zusammenfassung**

Erfindungsgemäß ist ein Verfahren zur Herstellung einer pharmazeutischen Zusammensetzung, umfassend 6-(4-Chlorphenyl)-2,2-dimethyl-7-phenyl-2,3-dihydro-1H-pyrrolizin-5-yl-essigsäurecholinsalz der Formel und einen oder mehrere pharmazeutische Hilfsstoffe, wobei das Verfahren die Schritte (i) bis (v) in der folgenden Reihenfolge umfasst
(i) Bereitstellen einer Mischung umfassend das Licofelon-Salz der Formel (I) und einen oder mehrere pharmazeutische Hilfsstoffe in Form eines homogenen Pulvers;
(ii) Verdichten des homogenen Pulvers zu einem Kompaktat;
(iii) Überführen des Kompaktates in ein Granulat;
(iv) optional das Mischen des Granulates mit weiteren Hilfsstoffen, und
(v) anschließendes Verpressen des Granulats zu Tabletten;
wobei die Schritte (i) bis (v) unter trockenen Bedingungen und bei Temperaturen von bis zu 40 °C, bevorzugt bis zu 30 °C, besonders bevorzugt bei Temperaturen von 24 bis 26 °C, optimal bei 25 °C durchgeführt werden.

## Beschreibung

Die vorliegende Erfindung betrifft ein Verfahren zur Herstellung einer stabilen pharmazeutischen Zusammensetzung umfassend 6-(4-Chlorphenyl)-2,2-dimethyl-7-phenyl-2,3-dihydro-1H-pyrrolizin-5-yl-essigsäurecholinsalz der Formel I:

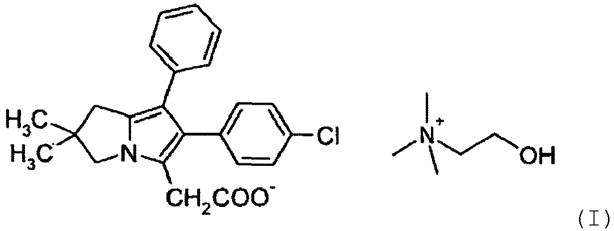

### Hintergrund der Erfindung

Licofelon - [2,2-Dimethyl-6-(4-chlorphenyl)-7-phenyl-2,3-dihydro-1H-pyrrolizin-5-yl]essigsäure - ist als nicht selektiver, dualer Inhibitor von Cyclooxygenase und 5-Lipoxygenase mit analgetischer, antipyretischer und antiinflammatorischer Aktivität Gegenstand zahlreicher Publikationen (vgl. WO 2005/041920).

WO 2005/041920 beschreibt, dass die Löslichkeit von Licofelon für eine zufriedenstellende Resorption ungenügend ist, und stellt fest, dass übliche Ansätze zur Verbesserung der Lösungsgeschwindigkeit schwerlöslicher fester Arzneistoffe für Licofelon nicht zum Ziel führten. So waren Versuche zur Vergrößerung der spezifischen Oberfläche durch Verringerung der Partikelgröße und die Erhöhung der Wirkstoff-Löslichkeit durch Einsatz von Tensiden im Falle von Licofelon mit starken Nachteilen verbunden. Vor diesem Hintergrund wählten die Erfinder der WO 2005/041920 zur Verbesserung der Lösungsgeschwindigkeit von Licofelon einen anderen Weg und beschreiben den Einsatz bestimmter hydrophiler Polymere bzw. hydrophiler Polyole. Ausweislich der WO 2005/041920 erlaubt der Einsatz hydrophiler Polymere bzw. Polyole die Bereitstellung fester pharmazeutischer Formulierungen mit besonders vorteilhaften Lösungseigenschaften von Licofelon.

Zur Herstellung der Licofelon-Formulierung wählt die WO 2005/041920 ein Verfahren, in dem zunächst eine Vormischung einzelner Komponenten bereitgestellt wird - beispielsweise in einem Wirbelschichtgranulator - bevor eine wässrige Lösung des hydrophilen Polymers auf diese Vormischung aufgesprüht wird. Das so erhaltene feuchte Granulat wird anschließend getrocknet, mit weiteren Füllstoffen versetzt und zu Tabletten verpresst.

Ein alternativer Ansatz zur Erhöhung der Löslichkeit von Licofelon ist der Einsatz bestimmter Salze. Die EP 2 350 082 B1 hebt in diesem Zusammenhang die Tromethamin- und Cholin-Salze von Licofelon hervor und beschreibt auch das Stabilitätsverhalten dieser Salze bei unterschiedlichen Lagerbedingungen. So wird beschrieben, dass die Cholin- und Tromethaminsalze von Licofelon bei erhöhten Temperaturen stabiler sind als die freie Säure (Licofelon). Insbesondere zeigen die Licofelontromethamin- und Licofeloncholinsalze eine verbesserte Stabilität auch unter verschärften Lagerbedingungen bei erhöhter Luftfeuchte.

Bei der Herstellung pharmazeutischer Zusammensetzungen von Licofelon-Salzen waren insofern keine Stabilitätsprobleme des Wirkstoffes zu erwarten. Einerseits beschreibt der Stand der Technik die erfolgreiche Herstellung Licofelon-haltiger pharmazeutischer Zusammensetzungen für die freie Säure (Licofelon), ohne dass Abbauprodukte des Wirkstoffes beobachtet wurden. Andererseits offenbart die Stabilitätsdiskussion von Licofelon eine *erhöhte* Stabilität der Licofelon-Salze.

Mit anderen Worten stand zu vermuten, dass es auch während der Herstellung von pharmazeutischen Zusammensetzungen umfassend das Cholinsalz von Licofelon zu keinen Stabilitätsproblemen des Wirkstoffes kommen sollte und dass die Bereitstellung entsprechender Zusammensetzungen in Analogie zur freien Säure realisierbar ist. Ein solches Verfahren wird in den Beispielen der WO 2005/041920 offenbart und arbeitet für kurze Zeiträume bei einer maximalen Temperatur von 60 °C.

Die vorliegenden Erfinder haben überraschenderweise gefunden, dass das für Licofelon (d.h. die freie Säure) in der WO 2005/041920 vorgeschlagene Verfahren nicht auf die diskutierten Salze übertragbar ist. Denn unter den in der WO 2005/041920 beschriebenen Bedingungen kommt es bei der Herstellung des Produktes zu unerwarteten Zersetzungsreaktionen, die beim Licofelon Wirkstoff selbst nicht auftreten. Tatsächlich haben weitere Untersuchungen der vorliegenden Erfinder ergeben, dass mit dem Cholin-Salz von Licofelon unerwartete Schwierigkeiten einhergehen, die sich insbesondere in der großtechnischen Herstellung der pharmazeutischen Zusammensetzungen zeigen. Im Falle des Licofelon-Cholinsalzes kommt es bereits während der Herstellung der pharmazeutischen Formulierungen unter den Routinebedingungen des Standes der Technik zu Abbaureaktionen, die aufwendige Qualitätskontrollen der fertigen Arzneiform erforderlich machen. Überraschenderweise setzen sich die Abbaureaktionen des Wirkstoffes mitunter noch in der fertigen Arzneiform fort. Daraus folgt, dass die im Vergleich zur freien Säure (Licofelon) im Stand der Technik offenbarte erhöhte Stabilität der reinen Salze nicht auf die fertige Arzneiform übertragbar ist.

Vor diesem Hintergrund zielt die vorliegende Erfindung darauf ab, ein verbessertes Verfahren zur Herstellung einer pharmazeutischen Zusammensetzung umfassend 6-(4-Chlorphenyl)-2,2-dimethyl-7-phenyl-2,3-dihydro-1H-pyrrolizin-5-yl-essigsäurecholinsalz der Formel I 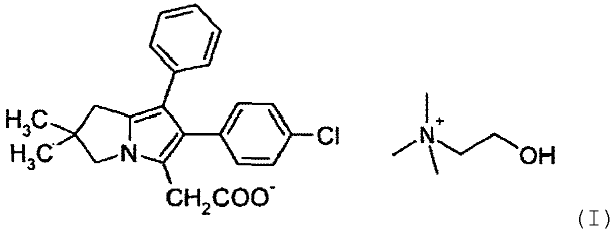 bereitzustellen.

### Beschreibung der Erfindung

Die vorliegende Erfindung offenbart daher ein Verfahren zur Herstellung einer pharmazeutischen Zusammensetzung, umfassend 6-(4-Chlorphenyl)-2,2-dimethyl-7-phenyl-2,3-dihydro-1H-pyrrolizin-5-yl-essigsäurecholinsalz der Formel I: 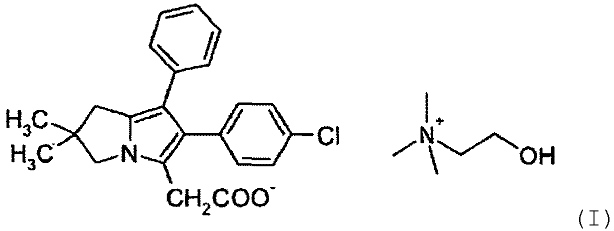 und einen oder mehrere pharmazeutische Hilfsstoffe, wobei das Verfahren die Schritte (i) bis (v) in der folgenden Reihenfolge umfasst
(i) Bereitstellen einer Mischung umfassend das Licofelon-Salz der Formel (I) und einen oder mehrere pharmazeutische Hilfsstoffe in Form eines homogenen Pulvers;
(ii) Verdichten des homogenen Pulvers zu einem Kompaktat;
(iii) Überführen des Kompaktates in ein Granulat;
(iv) optional das Mischen des Granulates mit weiteren Hilfsstoffen, und
(v) anschließendes Verpressen des Granulats zu Tabletten;
wobei die Schritte (i) bis (v) unter trockenen Bedingungen und bei Temperaturen von bis zu 40 °C, bevorzugt bis zu 30 °C, besonders bevorzugt bei Temperaturen von 24 bis 26 °C, optimal bei 25 °C durchgeführt werden.

In einem weiteren Aspekt betrifft die Erfindung pharmazeutische Zusammensetzungen, die nach dem Verfahren der vorliegenden Erfindung erhältlich sind. Ferner beschrieben wird die Verwendung der pharmazeutischen Zusammensetzung bei der Behandlung rheumatischer Erkrankungen.

Erfindungsgemäß werden in den hierin beschrieben Verfahren zur Herstellung einer pharmazeutischen Zusammensetzung, umfassend 6-(4-Chlorphenyl)-2,2-dimethyl-7-phenyl-2,3-dihydro-1H-pyrrolizin-5-yl-essigsäurecholinsalz der Formel I: 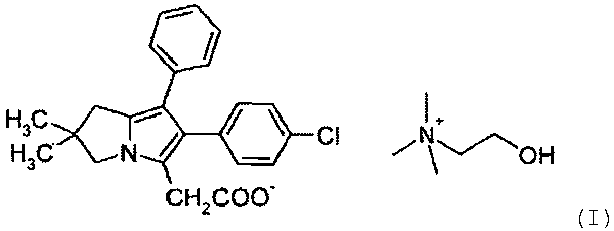 zunächst (i) das Licofelon-Salz der Formel (I) und ein oder mehrere pharmazeutische Hilfsstoffe als Mischung in Form eines homogenen Pulvers bereitgestellt; bevor (ii) dieses Pulver zu einem Kompaktat verdichtet wird. In einem dritten Schritt (iii) wird das so erhaltene Kompaktat schließlich in ein Granulat überführt. Dieses Granulat kann in einem optionalen Schritt (iv) mit einem oder mehreren weiteren Hilfsstoffen vermischt werden, bevor es in Schritt (v) zu Tabletten verpresst wird. Bei den Tabletten handelt es sich bevorzugt um Mikrotabletten. Die Tabletten, bevorzugt die Mikrotabletten, können bei Bedarf beschichtet werden.

Überraschenderweise wurde gefunden, dass die hierin beschriebene Reihenfolge der Schritte (i) bis (v) die Bereitstellung einer festen Arzneiform von Licofeloncholinsalz in Form von Tabletten erlaubt, ohne dass es bei der Herstellung oder der Lagerung der Tabletten zu nennenswerten Abbauprodukten des pharmazeutisch aktiven Wirkstoffes kommt.

Mit der vorliegenden Erfindung wird also ein Verfahren bereitgestellt, das zu einer stabilen pharmazeutischen Zusammensetzung umfassend das Cholinsalz von Licofelon führt, und das auch in großtechnischem Maßstab darstellbar ist. Insbesondere erlaubt das erfindungsgemäße Verfahren die Herstellung von pharmazeutischen Zusammensetzungen, bei deren Herstellung das Risiko und das Auftreten von Abbauprodukten deutlich verringert werden. Ferner sind die nach dem erfindungsgemäßen Verfahren hergestellten pharmazeutischen Zusammensetzungen deutlich stabiler als dies mit anderen Herstellungsverfahren der Fall ist. Das bedeutet, dass auch in der fertigen Zusammensetzung über längere Zeiträume von beispielsweise sechs bis sieben Monaten deutlich weniger Abbauprodukte des aktiven Wirkstoffes beobachtet werden, als dies für Tabletten der Fall ist, die nach Standardverfahren, beispielsweise gemäß der WO 2005/041920, hergestellt wurden.

Das erfindungsgemäße Verfahren geht dabei aus von einer Mischung umfassend das Licofelon-Salz der Formel (I) und einem oder mehreren pharmazeutischen Hilfsstoffen. Als Licofelon-Salz wird die chemische Verbindung gemäß Formel (I) bezeichnet, wobei die Reinheit der Verbindung gemäß Formel (I) in der homogenen Mischung bevorzugt bei 98 % oder mehr, bevorzugt bei 99 % oder mehr, liegt. Unter "Reinheit" versteht die vorliegende Erfindung in diesem Zusammenhang den Anteil an Licofelon-Cholinsalz bezogen auf das Salz und dessen Abbauprodukte.

Pharmazeutische Hilfsstoffe im Sinne der vorliegenden Erfindung sind Füllstoffe (z.B. Lactose, Cellulose, Mannit, Calciumphosphat), Bindemittel (z.B. Stärke, Polyvinylpyrrolidone, mikrokristalline Cellulose, Celluloseether), Fliessregulierungsmittel (z.B. hochdisperses Siliziumdioxid), Formentrennmittel (z.B. Polyethylenglykole, Talkum, Magnesiumstearat, Stearinsäure, Stearylfumarat, Glycerylbehenat), und Zerfallsförderer (z.B. Natriumstärkeglycolat, Polyvinylpyrrolidone, (z.B. vernetzte) Carboxymethylcellulose).

Besonders geeignet zum Einsatz in dem erfindungsgemäßen Verfahren sind Füllstoffe und Bindemittel (z.B. Lactose, mikrokristalline Cellulose, silifizierte mikrokristalline Cellulose, Dicalciumphosphat), Fliessregulierungsmittel (z.B. hochdisperses Siliziumdioxid, amorphe mikronisierte Kieselsäure), Formentrennmittel (z.B. Magnesiumstearat, Stearylfumarat, Glycerylbehenat), und Zerfallsförderer (z.B. quervernetzte Polyvinylpyrrolidone).

Die Mischung umfassend das Licofelon-Salz und die pharmazeutischen Hilfsstoffe wird in Form eines homogenen Pulvers bereitgestellt, das anschließend zu einem Kompaktat verdichtet wird. Die homogene Mischung zur Kompaktierung kann nach Herstellung einer Vormischung bereitgestellt werden. In einer besonders bevorzugten Ausführungsform der Erfindung erfolgt der Schritt (ii), das heißt das Verdichten des homogenen Pulvers zu dem Kompaktat, in einer Kompaktiereinheit, in der zwei sich gegenseitig drehende Walzen die Vormischung mit einer bestimmten Kraft kompaktieren. Dadurch wird eine Verdichtung der Kompaktiermischung auf ca. die Hälfte des ursprünglichen Volumens erreicht. Die Verdichtung ist abhängig vom Wirkstoffanteil (dem Anteil des Licofelon-Cholinsalzes in der Mischung), der zwischen 10 bis 90 Gewichtsprozent, bezogen auf das Gesamtgewicht der Mischung, liegen kann. Typische Wirkstoffanteile liegen beispielsweise zwischen 20 und 80, bevorzugt zwischen 30 und 60 Gewichtsprozent Licofelon-Cholinsalz, bezogen auf die gesamten Bestandteile der Mischung.

In einer bevorzugten Ausführungsform der Erfindung wird das homogene Pulver zum Verdichten in Schritt (ii) zwischen zwei Walzen hindurch geleitet. Eine besonders geeignete Variante des erfindungsgemäßen Verfahrens greift auf Walzenkompaktoren zurück, z.B. Alexanderwerk Roller Compactors (BT, WP), Vector Roller Compactors (TFC), oder Gerteis Walzenkompaktoren (MP, UP). Die benötigte Kraft liegt bevorzugt in einem Bereich von 2 bis 20, bevorzugt 2 bis 12, besonders bevorzugt 2-10, und ganz besonders bevorzugt 2-6 kN/cm, angegeben als Verhältnis zur Walzenbreite. Der Pressspalt zwischen den Walzen beträgt bevorzugt 1 - 6mm. Idealerweise kommen Walzen mit einer Walzenbreite von 20 - 120mm, einer Durchsatzleistung bis 400kg/h und einer Rotationsgeschwindigkeit von 1 - 38 upm zum Einsatz.

In dem darauf folgenden Schritt (iii) wird das erhaltene Kompaktat in ein Granulat überführt. Hierzu kann das Kompaktat beispielsweise durch ein Sieb granuliert werden. Geeignete Siebe haben eine Maschenweite von 0,6 bis 1,4 mm. Hierdurch wird gewährleistet, dass die Oberkorngröße des Granulats 1,4 mm nicht überschreitet.

Die Erfinder gehen davon aus, dass das so erhaltene Granulat besonders gut für die Herstellung stabiler pharmazeutischen Zusammensetzungen umfassend das Cholinsalz von Licofelon geeignet ist. Das Licofelon Cholinsalz kann auf diese Weise trocken und mit geringer Licht- und Sauerstoffexposition verarbeitet werden. Des Weiteren führt die verringerte Dichte des Granulats zu einer besseren Tablettierbarkeit.

In dem optionalen Schritt (iv) werden bei Bedarf weitere Hilfsstoffe hinzugegeben. Nach dem Mischen des Granulates mit diesen optionalen weiteren Hilfsstoffen wird anschließend in Schritt (v) das erhaltene Granulat zu Tabletten verpresst.

Erfindungsgemäß werden die Schritte (i) bis (v) unter trockenen Bedingungen durchgeführt. Der Ausdruck "trockene" Bedingungen im Sinne der vorliegenden Erfindung bedeutet, dass eine relative Feuchte der Umgebungsatmosphäre von 75 % , bevorzugt von 65 %, besonders bevorzugt von 55 % nicht überschritten wird. Besonders bevorzugt werden die Hilfsstoffe in trockener Form eingesetzt, so dass der Wassergehalt der Tablette 7 Gewichtsprozent, bevorzugt 5 Gewichtsprozent, bevorzugter 4 Gewichtsprozent, besonders bevorzugt 3,5 Gewichtsprozent nicht überschreitet.

### Beispiele

Es wurden Tabletten nach folgendem Verfahren hergestellt: 60% Licofelon Cholinsalz wurden mit silifizierter mikrokristalliner Cellulose, hochdispersem Siliziumdioxid, Magnesiumstearat und quervernetztem Polyvinylpyrrolidone nach Vormischung trocken granuliert (Walzenkompaktierung). Nach Siebung und Endmischung wurden aus dem Granulat Tabletten gepresst.

Die so erhaltenen Tabletten wurden unmittelbar nach ihrer Herstellung auf Abbauprodukte von Licofelon untersucht. Die erhaltenen Tabletten wurden bei 40°C/75%RF gelagert. Auch nach 7 Monaten ergaben sich keine Hinweise auf einen Anstieg der Abbauprodukte des aktiven Wirkstoffes über die gemäß allgemein verbindlichen Industriestandards einzuhaltenden Grenzwerte hinaus.

## Patentansprüche

1. Verfahren zur Herstellung einer pharmazeutischen Zusammensetzung, umfassend 6-(4-Chlorphenyl)-2,2-dimethyl-7-phenyl-2,3-dihydro-1H-pyrrolizin-5-yl-essigsäurecholinsalz der Formel I: 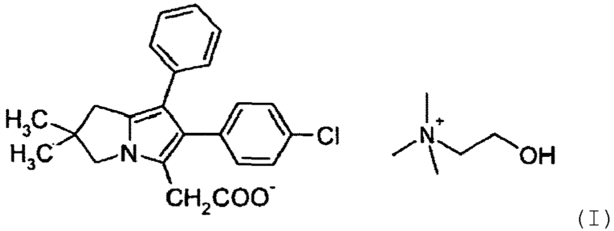 und einen oder mehrere pharmazeutische Hilfsstoffe, wobei das Verfahren die Schritte (i) bis (v) in der folgenden Reihenfolge umfasst
(i) Bereitstellen einer Mischung umfassend das Licofelon-Salz der Formel (I) und einen oder mehrere pharmazeutische Hilfsstoffe in Form eines homogenen Pulvers;
(ii) Verdichten des homogenen Pulvers zu einem Kompaktat;
(iii) Überführen des Kompaktates in ein Granulat;
(iv) optional das Mischen des Granulates mit weiteren Hilfsstoffen, und
(v) anschließendes Verpressen des Granulats zu Tabletten;
wobei die Schritte (i) bis (v) unter trockenen Bedingungen und bei Temperaturen von bis zu 40 °C, bevorzugt bis zu 30 °C, besonders bevorzugt bei Temperaturen von 24 bis 26 °C, optimal bei 25 °C durchgeführt werden.

2. Verfahren nach Anspruch 1, wobei die Schritte (i) bis (v) bei Temperaturen von 30 °C, bevorzugt bei Temperaturen von 24 bis 26 °C durchgeführt werden.

3. Verfahren nach einem der Ansprüche 1 und 2, wobei Schritt (ii) und (iii) mit einem Walzenkompaktor durchgeführt werden.

4. Verfahren nach einem der vorhergehenden Ansprüche, wobei die Tabletten in Schritt (v) Mikrotabletten sind.

5. Verfahren nach einem der vorhergehenden Ansprüche, wobei die Hilfsstoffe in Schritt (ii) und in dem optionalen Schritt (iv) ausgewählt werden aus Füllstoffen, Bindemitteln, Fliessregulierungsmitteln, Formentrennmitteln und Zerfallsförderern.

6. Verfahren nach Anspruch 5, wobei die Füllstoffe ausgewählt sind aus der Gruppe bestehend aus Lactose, Cellulose, Mannit und Calciumphosphat; und/oder wobei die Bindemittel ausgewählt sind aus der Gruppe bestehend aus Stärke, Polyvinylpyrrolidone, mikrokristalliner Cellulose und Celluloseethern; und/oder wobei das Fliessregulierungsmittel hochdisperses Siliziumdioxid ist; und/oder wobei die Formentrennmittel ausgewählt sind aus der Gruppe bestehend aus Polyethylenglykolen, Talkum, Magnesiumstearat, Stearinsäure, Stearylfumarat und Glycerylbehenat; und/oder wobei der Zerfallsförderer ausgewählt ist aus der Gruppe bestehend aus Natriumstärkeglycolat, Polyvinylpyrrolidon, und Carboxymethylcellulose.

7. Verfahren nach einem der vorhergehenden Ansprüche, wobei die relative Feuchte der Umgebungsatmosphäre beim Durchführen der Schritte (i) bis (v) einen Wert von 75 % nicht überschreitet.

8. Verfahren nach einem der vorhergehenden Ansprüche, wobei die Oberkorngröße des in Schritt (iii) erhaltenen Granulats 1,4 mm nicht überschreitet.

9. Pharmazeutische Zusammensetzung, erhältlich nach dem Verfahren der Ansprüche 1 bis 8.

10. Pharmazeutische Zusammensetzung nach Anspruch 9 zur Verwendung bei der Behandlung von rheumatischen Erkrankungen.
